# EUROPÄISCHE PATENTANMELDUNG

(11) **EP 1 764 040 A2**
(43) Veröffentlichungstag der Anmeldung: **21.03.2007**
(21) Anmeldenummer: 06119666.3
(22) Anmeldetag: 29.08.2006
(51) Int. Cl.: A61B 6/08

(54) **Verfahren zur artefaktreduzierten radiologischen 3D-Bildgebung, Medizinische Bildgebungsvorrichtung und Verfahren zur Erstellung eines Therapieplans**

(30) Priorität: 16.09.2005 DE 102005044407; 16.09.2005 US 717831
(71) Anmelder: SIEMENS AKTIENGESELLSCHAFT, 80333 München (DE)
(72) Erfinder: Rietzel, Eike Dr., 64289, Darmstadt (DE)

(57) **Zusammenfassung**

In einem Verfahren zur artefaktreduzierten radiologischen 3D-Bildgebung eines Bildgebungsvolumens einer medizinischen Bildgebungsvorrichtung weist das Bildgebungsvolumen mindestens ein einen Artefakt verursachendes Störelement auf. Dazu wird ein Patient in einer ersten Bildgebungsposition in der Bildgebungsvorrichtung positioniert und eine erste 3D-Bildgebung mit einem ersten Satz Durchstrahlungsschichten in dieser ersten Bildgebungsposition durchgeführt, wobei das Störelement innerhalb einer ersten Durchstrahlungsschicht des ersten Satzes liegt und eine Rohdatengewinnung in dieser ersten Durchstrahlungsschicht beeinflusst. Ein dabei gewonnener erster Rohdatensatz des Bildgebungsvolumens, der artefaktbeeinflussten Rohdaten für die erste Durchstrahlungsschicht aufweist, wird bereitgestellt. Ferner wird der Patient in einer zweiten Bildgebungsposition positioniert, welche eine, sich von der räumlichen Lage der ersten Bildgebungsposition unterscheidende, Lage bezüglich der Bildgebungsvorrichtung aufweist. In dieser zweiten Bildgebungsposition wird eine zweite 3D-Bildgebung mit einem zweiten Satz Durchstrahlungsschichten durchgeführt, wobei das Störelement innerhalb einer zweiten Durchstrahlungsschicht des zweiten Satzes liegt und eine Rohdatengewinnung in dieser zweiten Durchstrahlungsschicht beeinflusst, wobei die zweite Durchstrahlungsschicht die erste Durchstrahlungsschicht in einem Schnittvolumen schneidet, in dem das Störelement liegt. Ein dabei gewonnener zweiter Rohdatensatz des Bildgebungsvolumens wird bereitgestellt, der artefaktbeeinflussten Rohdaten für die zweite Durchstrahlungsschicht und nicht artefaktbeeinflusste Rohdaten für die erste Durchleuchtungsschicht außerhalb der Schnittlinienvolumens aufweist. Durch Kombinieren der gewonnen Rohdatensätze zur Berechnung eines artefaktreduzierten 3D-Bilddatensatzes des Bildgebungsvolumens und/oder Berechnen eines ersten 3D-Bilddatensatzes aus dem ersten Rohdatensatz, Berechnen eines zweiten 3D-Bilddatensatzes aus dem zweiten Rohdatensatz, Kombinieren des ersten und des zweiten 3D-Bilddatensatzes zu einem artefaktreduzierten 3D-Bilddatensatz wird der artefaktreduzierte 3D-Datensatz erzeugt.

## Beschreibung

Die Erfindung betrifft ein Verfahren zur artefaktreduzierten radiologischen 3D-Bildgebung eines Bildgebungsvolumens einer medizinischen Bildgebungsvorrichtung, wobei das Bildgebungsvolumen mindestens ein einen Artefakt verursachendes Störelement aufweist. Ferner betrifft die Erfindung eine medizinische Bildgebungsvorrichtung zur Erzeugung eines radiologischen 3D-Bilddatensatzes sowie ein Verfahren zur Erstellung eines Therapieplans zum Bestrahlen eines Patienten mit Partikeln einer Partikeltherapieanlage.

Bei der radiologischen Bildgebung mit z.B. einem Computertomographiegerät (CT-Gerät) führen Materialien mit hoher Dichte, z.B. Zahnfüllungen oder Hüftprothesen, zu einer kompletten Absorption der der Bildgebung zugrunde liegenden Röntgenstrahlung. Derartige Störelemente im zu durchstrahlenden Bereich eines Patienten bewirken somit den Verlust von auswertbaren Projektionsdaten. Somit wird die Rekonstruktion eines Bilddatensatzes aus derartigen Rohdaten für anatomische Bereiche, die in Durchstrahlungsrichtung vor und hinter den Störelementen mit hoher Dichte liegen, ungenau und nicht mehr aussagekräftig. Die Auswirkungen aufgrund einer mangelhaft durchführbaren Rekonstruktion in einem 3D-Bilddatensatz werden als sog. Metallartefakte bezeichnet. Die Auswirkungen beschränken sich nicht nur auf die Region des Materials mit der hohen Dichte, sondern wirken sich auf alle Durchstrahlungsschichten aus, in denen das Störelement liegt.

Eine derart eingeschränkte radiologische Bildgebung wirkt sich insbesondere auf die Genauigkeit einer Therapieplanung bei einer Strahlentherapie, insbesondere bei einer Partikeltherapie, aus. Bei einer Therapieplanung erfolgt eine genaue Dosis und/oder Reichweitenberechnung anhand der bei der CT-Bildgebung gemessenen Dichteverteilung. Entscheidend für die Reichweitenberechnung und damit für die Genauigkeit der Dosisverteilung ist die Verfügbarkeit einer sehr genauen Dichteverteilung des Gewebes, gemessen in HU-Einheiten (HoundsfieldUnit HU) als Maß für die Schwächung von Röntgenstrahlung. Denn die lokale Schwächung der Strahlung im Patienten wird mittels der aus einer Bestrahlungsplanungs-CT-Aufnahme gewonnenen HU-Werte berechnet. Eine verbesserte, d.h. artefaktreduzierte, Bildgebung wirkt sich somit positiv auf die Genauigkeit bei der Therapieplanung aus.

Üblicherweise werden bei der Bildgebung und Therapieplanung Schichten und Winkelbereiche, in denen eine komplette Absorption der Durchstrahlung aufgetreten ist, in den Bildgebungsdatensätzen vor der Rekonstruktion ermittelt und mit umliegenden Projektionsdaten/Rohdaten interpoliert. Dabei wird darauf geachtet, die Projektionsdaten möglichst realistisch zu vervollständigen. Eine andere Vorgehensweise, die zur Zeit in der Strahlentherapie angewandt wird, ist es, die HU-Werte im CT-Bildgebungsbereich, die von Metallartefakten betroffen sind, manuell auf einigermaßen sinnvolle Wert zu setzen. Trotzdem werden oft Einstrahlrichtungen der Therapiestrahlen/Therapiepartikelstrahlen, in denen nicht zu vernachlässigende Metallartefakte aufgetreten sind, für eine Bestrahlung ausgeschlossen. Alternativ können beispielsweise derartige Störelemente mechanisch entfernt oder, wie z.B. bei Zahnfüllungen, mit nicht störenden Materialien aus z.B. Kunststoff ersetzt werden.

Eine Aufgabe der Erfindung ist es, die Gewinnung von möglichst artefaktreduzierten Bilddaten bei der radiologischen Bildgebung zu ermöglichen. Eine weitere Aufgabe ist es, einen Bilddatensatz für die Therapieplanung bereitzustellen, der eine exakte Dosis und/oder Reichweitenberechnung auch in Regionen erlaubt, die von Störelementen betroffen sind.

Die Aufgabe bezogen auf das Verfahren zur artefaktreduzierten radiologischen 3D-Bildgebung wird durch das Verfahren nach Anspruch 1 gelöst. Ferner wird die Aufgabe durch eine medizinische Bildgebungsvorrichtung nach Anspruch 5 gelöst. Die Aufgabe bezogen auf die Erstellung eines Therapieplans wird durch ein Verfahren zur Erstellung eines Therapieplans nach Anspruch 7 gelöst.

Ein Vorteil der Erfindung liegt darin, dass die Bestimmung von HU-Werten von Gewebe eines Patienten verbessert wird. D.h., die artefaktbetroffenen Regionen eines Bilddatensatzes sind in ihrer Ausdehnung im Vergleich zu konventionellen 3D-Bilddatensätzen reduziert. Dies wirkt sich bei der Therapieplanung auf eine Verbesserung der Reichweitenberechnung und damit auf eine Erhöhung der Präzision bei der Bestrahlung aus. In der Diagnostik können aufgrund der Reduzierung von metallartefaktbetroffenen Regionen auch anatomische Bereiche beurteilt werden, die zuvor aufgrund der Artefakte nicht sichtbar waren.

In einer Ausführungsform der Erfindung erfolgt die Datengewinnung eines zu scannenden Objekts mit zwei oder mehreren Bildgebungsschritten, die unterschiedliche Kippstellungen einer CT-Gantry aufweisen. In den verschiedenen Bildgebungsvorgängen bilden sich die artefaktbetroffenen Regionen in unterschiedlich räumlich angeordneten Durchstrahlungsschichten aus. Neben einer Kippung, Verschiebung oder Drehung der beispielsweise CT-Gantry (allgemein der Bildgebungsvorrichtung) kann das zu scannende Untersuchungsobjekt zusätzlich relativ zum beispielsweise CT-Gerät verschoben, gedreht oder gekippt werden. Letzteres bewirkt ebenfalls eine Änderung der artefaktbetroffenen Regionen im Bildgebungsvolumen.

Bei einem derartigen Vorgehen liegen die aufgrund der beispielsweise vollständigen Absorption der Röntgenstrahlung nicht mehr rekonstruierbare Bereich bei den verschiedenen Rekonstruktionen an unterschiedlichen Positionen; d.h., es sind unterschiedliche Rohdaten oder Bilddatenelemente von den Störelementen betroffen. Mit diesen Daten bestimmt man in allen rekonstruierten Bildgebungsvolumen die Bereiche, die von den Störelementen beeinflusst wurden. Ihre relative Lage zueinander ist durch die Einstellung bei der Bildgebung, z.B. relativer Kippwinkel der Kippestellungen, eindeutig bekannt. Durch eine Kombination von Bildgebungsvolumenelementen aus den verschiedenen Bildgebungen, lässt sich dann ein Bilddatensatz gewinnen, der weniger bzw. sogar gar keine durch die Störelemente bedingte Artefakte aufweist.

In einer alternativen Ausführungsform wird schon die Rekonstruktion mit einem modifizierten Rekonstruktionsalgorithmus durchgeführt, der die Rohdaten der verschiedenen Projektionen bereits vor der Rekonstruktion des Bilddatensatzes im Hinblick auf artefaktbedingte Störungen untersucht und derartige Bildstörungen durch eine entsprechende Kombination der Projektionsrohdaten für das zu berechnende Bild minimiert.

Die den verschiedenen Bildgebungen zugrunde liegenden Bildgebungspositionen können durch eine Verschiebung, Verkippung oder Verdrehung des Patienten und/oder der Bildgebungsvorrichtung bewirkt werden. Möglichst unterscheiden sich die Bildgebungspositionen derart, dass sich die Einflüsse der Artefakte auf möglichst unterschiedliche und nur wenig überlappende Bildgebungsvolumenbereiche beschränken. Dies hat den Vorteil, dass der Einfluss der Störelemente im Idealfall bis auf das Störelement selbst reduziert werden kann.

In einer Ausführungsform des Verfahrens zur Erstellung eines Therapieplans für die Strahlentherapie, insbesondere zum Bestrahlen eines Patienten mit Partikeln einer Partikeltherapieanlage, wird zuerst ein möglichst artefaktreduziertes radiologisches Bild mit Hilfe der Bildgebung von räumlich unterschiedlich angeordneten Bildgebungsvolumen erzeugt. Dieser artefaktreduzierte radiologische Bilddatensatz dient dann als Grundlage für die Bestimmung von Bestrahlungsparametern wie Bestrahlungsbereich, Dosisverteilung, Partikelstrahlenergie und/oder Partikelstrahlintensität.

Weitere vorteilhafte Ausführungsformen mit der Erfindung sind durch die Merkmale der Unteransprüche gekennzeichnet.

Es folgt die Erläuterung von mehreren Ausführungsbeispielen der Erfindung anhand der Figuren 1 bis 5. Es zeigen:
- Figur 1: eine beispielhafte Verdeutlichung eines erfindungsgemäßen Bildgebungsvorgangs und seiner Applikation,
- Figur 2: verschiedene Bildgebungspositionen eines zu scannenden Patienten,
- Figur 3 und 4: entsprechende Auswirkungen eines Metallelements auf die Bildgebung und
- Figur 5: die Gewinnung eines artefaktreduzierten Bildgebungsdatensatzes mittels der Bildgebungen aus Figur 3 und 4.

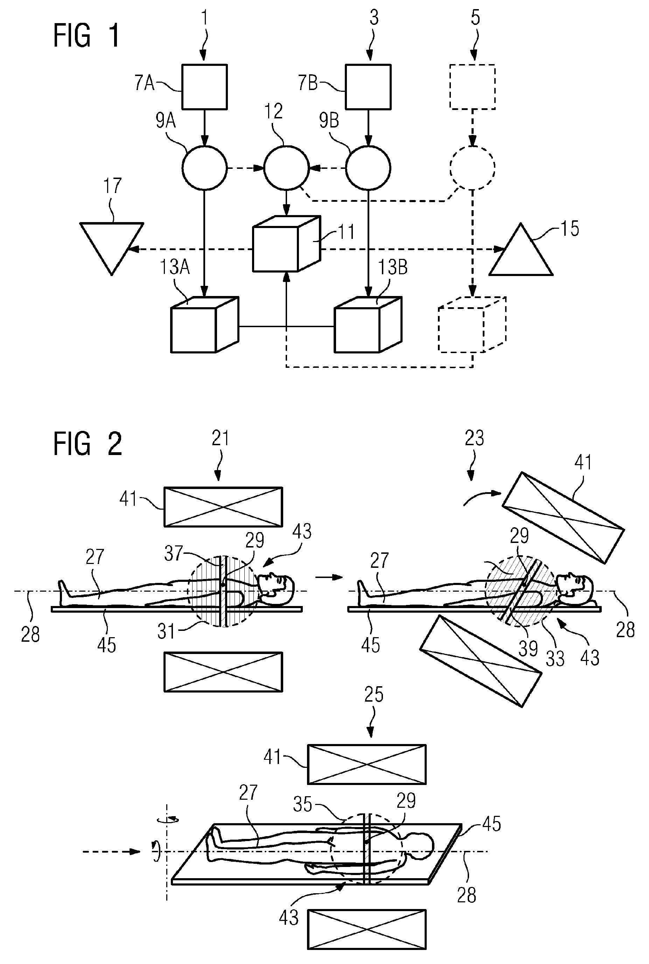

Figur 1 zeigt schematisch ein beispielhaftes Flussdiagramm zur Verdeutlichung der Vorgehensweise bei der Erzeugung von artefaktreduzierten Bilddaten. Das Verfahren wird anhand von zwei Bildgebungsvorgängen 1 und 3 verdeutlicht, wobei auch weitere Bildgebungsvorgänge 5 (punktiert angedeutet) einbezogen werden können. Jede der Bildgebungsvorgänge 1,3,5 beginnt mit einem Positioniervorgang, bei dem ein Patient in eine erste Bildgebungsposition 7A bzw. eine zweite Bildgebungsposition 7B innerhalb einer Bildgebungsvorrichtung positioniert wird (zur Verdeutlichung s. Figur 2 mit dazugehöriger Beschreibung). In den Bildgebungspositionen 7A, 7B wird jeweils eine 3D-Bildgebung durchgeführt, bei der jeweils ein Satz von Durchstrahlungsschichten mit einer Strahlungsquelle durchstrahlt und einem Strahlungsdetektor ortsaufgelöst aufgenommen wird. Dadurch wird ein erster Rohdatensatz 9A bzw. ein zweiter Rohdatensatz 9B erzeugt und zur weiteren Bearbeitung bereitgestellt. Entscheidend ist nun, dass sich die Bildgebungspositionen 7A und 7B voneinander in der geometrischen Anordnung zur Durchleuchtungsvorrichtung unterscheiden, denn nur dann liegt ein Störelement bei dem ersten Bildgebungsvorgang 1 in einer (oder in mehreren) ersten Durchstrahlungsschicht und beim Bildgebungsvorgang 3 in einer (oder mehreren) zweiten Durchstrahlungsschicht. Die Durchstrahlungsschichten schneiden sich in einem Schnittvolumen, in dem auch das Störelement liegt.

Zur weiteren Vorgehensweise zur Erzeugung eines möglichst artefaktreduzierten Bilddatensatzes 11 kann nun entweder eine Kombination der gewonnenen Rohdatensätze 9A und 9B genutzt werden. Zusätzlich oder alternativ kann die Berechnung mit Hilfe von 3D-Bilddatensätzen 13A und 13B, die jeweils aus den Rohdatensätzen 9A bzw. 9B berechnet wurden, erfolgen. Dazu werden beispielsweise Bilddatenelemente, die im Bilddatensatz 13B artefaktbeeinflusst sind, durch ihnen entsprechende Bilddatenelemente des Bilddatensatzes 13A, die nicht artefaktbeeinflusst sind, ersetzt.

Die Kombination der Rohdatensätze 9A und 9B kann beispielsweise einen Rohdatensatz 12 ergeben. Der Rohdatensatz 12 und/oder die beide Rohdatensätze 9A,9B können gemeinsam in einem entsprechend angepassten Rekonstruktionsalgorithmus zur Gewinnung des Bilddatensatzes 11 genutzt werden.

Der artefaktreduzierte 3D-Datensatz 11 erlaubt zum einen eine Diagnose 15 des zu untersuchenden Objekts auch in Bereichen, die bei konventioneller Bildgebung artefaktbelastet wären. Ferner ist es möglich, diesen Datensatz zur Therapieplanung 17 einer Strahlentherapie als Grundlage für die Berechnung der Strahlen- oder Partikelabsorption bei der Bestrahlung zu verwenden, um beispielsweise notwendige Dosisverteilungen, Strahlintensitäten, Partikelenergien oder Partikelintensitäten für die Bestrahlung festzulegen.

Figur 2 zeigt beispielhaft drei aufeinander folgende Bildgebungsvorgänge 21, 23 und 25 mit jeweils unterschiedlichen Bildgebungspositionen, d.h. mit unterschiedlichen geometrischen Anordnungen der Durchstrahlungsschichten in Bezug zum Patienten 27. Beim Bildgebungsvorgang 21 liegt der Patient 27 waagrecht auf dem Rücken, d.h., seine Körperlängsachse 28 weist z.B. einen Winkel von ca. 90° zur Durchleuchtungsrichtung auf.

Ferner erkennt man ein Störelement 29 -beispielsweise ein Herzschrittmacher, eine Zahnkrone, ein Metallimplantat etc.- sowie die aufgrund der Bildgebungsposition bedingt zum Patienten ausgerichteten und zueinander parallelen Sätze 31, 33, 35 von Durchstrahlungsschichten. Die jeweils vom Störelement 29 betroffene Durchstrahlungsschicht wurden gestrichelt gekennzeichnet, beispielsweise die Durchleuchtungsschicht 37 des ersten Bildgebungsvorgangs 21 und die Durchleuchtungsschicht 39 des Bildgebungsvorgangs 23.

Eine Bildgebungsvorrichtung 41 ist beispielsweise ein CT-Gerät, ein Cone-Beam-Röntgengerät oder ein sonstiges zur 3D-Bildgebung geeignetes Durchleuchtungsgerät. Es ist vorzugsweise drehbar, kippbar und verschiebbar ausgebildet, so dass in einem Bildgebungsvolumen 43 der Bildgebungsvorrichtung 41 die durchleuchteten Schichten verschieden ausgerichtet sind. Der Bildgebungsvorgang 23 wird mit der zur Senkrechten um einen Winkel verkippten Bildgebungsvorrichtung 41 durchgeführt, wobei die Verkippung in Richtung der Patientenlängsachse erfolgte. Die durchleuchteten Schichten sind entsprechend zur Waagrechten um einen Winkel verkippt.

Die Bildgebungsvorrichtung 41 weist eine Patientenlagerungsvorrichtung auf, beispielsweise einen Patiententisch 45 oder einen Patientenstuhl. Vorzugsweise erlaubt auch die Patientenhalterungsvorrichtung ein Drehen, Kippen und/oder Verschieben des Patienten 27, um ihrerseits verschiedene Bildgebungspositionen einstellen zu können. So wurde für den Bildgebungsvorgang 25 der Patient 27 sowohl um eine zur Körperlängsachse 28 parallele Achse als auch um eine senkrechte Achse rotiert.

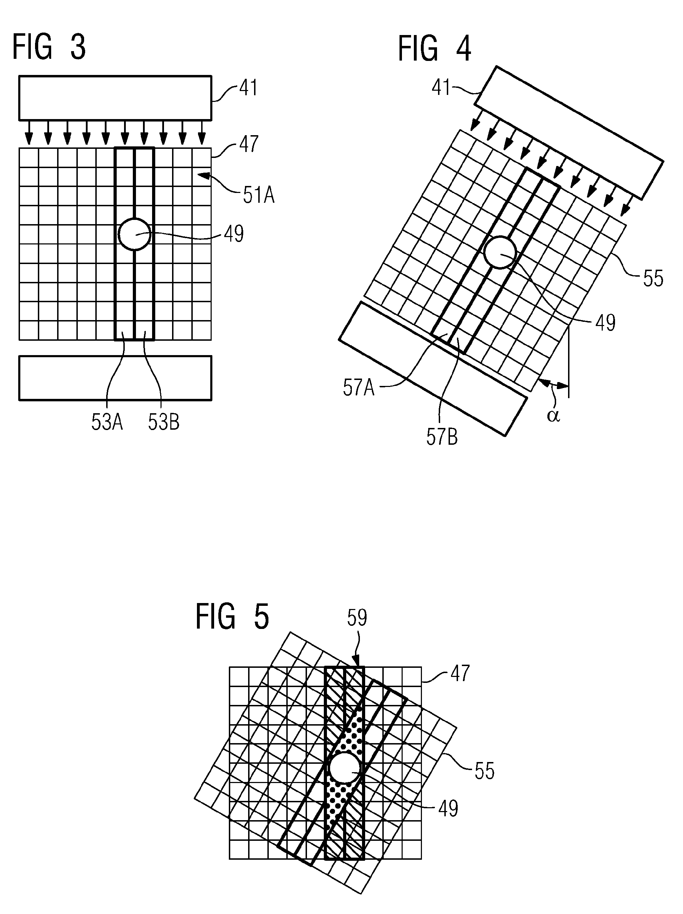

Figur 3 bezieht sich auf einen Bildgebungsvorgang ähnlich dem Bildgebungsvorgang 21 aus Figur 2 und zeigt einen Schnitt durch einen 3D-Bilddatensatz 47, in dem man ein Störelement 49 und eine durch Kästchen dargestellte Bildelementstruktur 51A mit mehreren senkrechten Durchleuchtungsschichten erkennt. Das Störelement 49 wirkt sich im Bilddatensatz auf zwei nebeneinander liegende Durchleuchtungsschichten 53A und 53B aus.

Figur 4 zeigt einen Schnitt durch einen Bilddatensatz 55, der z.B. durch einen Bildgebungsvorgang entsprechend dem Bildgebungsvorgang 23 aus Figur 2 hervorging. Die Ausrichtung der Durchleuchtungsschichten ist um einen Winkel α zu den senkrechten ausgerichteten Durchleuchtungsschichten in Figur 3 gedreht. Das Störelement 49 wirkt sich nun auf Durchleuchtungsschichten 57A und 57B aus.

Figur 5 zeigt eine Überlagerung der Bilddatensätze 47 und 55. Man erkennt, dass die vom Störelement 49 beeinflussten Bildelemente der Schichten 53A, 53B bzw. 57A, 57B nur in einem eingeschränkten Bereich überlappen. Nimmt man sich beispielsweise den Bilddatensatz 47 als Grundlage, kann man die Bildelemente im schraffierten Bereich 59 durch die entsprechenden Bildelemente des Bilddatensatzes 55 näherungsweise ersetzen.

Um die verbleibenden punktierten Bereiche mit gemessenen HU-Werten zu füllen, wird beispielsweise ein Bildgebungsvorgang entsprechend dem Bildgebungsvorgang 25 aus Figur 2 benötigt, der eine Drehung der Patientenliege um die Patientenlängsachse sowie eine Rotation um die Vertikalachse zugrunde liegt. Ein so korrigierter 3D-Bilddatensatz trägt den hohen Anforderungen beispielsweise in der Strahlentherapie genüge.

## Patentansprüche

1. Verfahren zur artefaktreduzierten radiologischen 3D-Bildgebung eines Bildgebungsvolumens einer medizinischen Bildgebungsvorrichtung, wobei das Bildgebungsvolumen mindestens ein einen Artefakt verursachendes Störelement aufweist, mit folgenden Verfahrensmerkmalen:
- Positionieren eines Patienten in einer ersten Bildgebungsposition in der Bildgebungsvorrichtung,
- Durchführen einer ersten 3D-Bildgebung mit einem ersten Satz Durchstrahlungsschichten in dieser ersten Bildgebungsposition, wobei das Störelement innerhalb einer ersten Durchstrahlungsschicht des ersten Satzes liegt und eine Rohdatengewinnung in dieser ersten Durchstrahlungsschicht beeinflusst,
- Bereitstellen eines ersten Rohdatensatzes des Bildgebungsvolumens, der artefaktbeeinflussten Rohdaten für die erste Durchstrahlungsschicht aufweist,
- Positionieren des Patienten in einer zweiten Bildgebungsposition, welche eine, sich von der räumlichen Lage der ersten Bildgebungsposition unterscheidende, Lage bezüglich der Bildgebungsvorrichtung aufweist,
- Durchführen einer zweiten 3D-Bildgebung mit einem zweiten Satz Durchstrahlungsschichten in dieser zweiten Bildgebungsposition, wobei das Störelement innerhalb einer zweiten Durchstrahlungsschicht des zweiten Satzes liegt und eine Rohdatengewinnung in dieser zweiten Durchstrahlungsschicht beeinflusst, wobei die zweite Durchstrahlungsschicht die erste Durchstrahlungsschicht in einem Schnittvolumen schneidet, in dem das Störelement liegt,
- Bereitstellen eines zweiten Rohdatensatzes des Bildgebungsvolumens, der artefaktbeeinflussten Rohdaten für die zweite Durchstrahlungsschicht und nicht artefaktbeeinflusste Rohdaten für die erste Durchleuchtungsschicht außerhalb der Schnittlinienvolumens aufweist,
- Erzeugen eines artefaktreduzierten 3D-Datensatzes durch:
a) Kombinieren der gewonnen Rohdatensätze zur Berechnung eines artefaktreduzierten 3D-Bilddatensatzes des Bildgebungsvolumens und/oder
b) Berechnen eines ersten 3D-Bilddatensatzes aus dem ersten Rohdatensatz, Berechnen eines zweiten 3D-Bilddatensatzes aus dem zweiten Rohdatensatz, Kombinieren des ersten und des zweiten 3D-Bilddatensatzes zu einem artefaktreduzierten 3D-Bilddatensatz.

2. Verfahren nach Anspruch 1, wobei die zweite Bildgebungsposition durch eine Verschiebung, Verkippung und/oder Verdrehung des Patienten und/oder der Bildgebungsvorrichtung bewirkt wird.

3. Verfahren nach Anspruch 1 oder 2, wobei beim Kombinieren des ersten und des zweiten 3D-Bilddatensatzes ein artefaktbeeinflusste Bilddateneintrag des ersten oder zweiten Bilddatensatzes mit einem nicht artefaktbeeinflussten Bilddateneintrag des zweiten bzw. ersten Bilddatensatzes ersetzt wird.

4. Verfahren nach einem der Ansprüche 1 bis 3, wobei ein oder mehrere weitere Bildgebungen in weiteren unterschiedlichen Bildgebungspositionen gewonnen und zur Erzeugung des artefaktreduzierten 3D-Bilddatensatz benutzt werden, wodurch die Anzahl der ersetzbaren artefaktbeeinflussten Bilddateneinträge vergrößert wird.

5. Medizinische Bildgebungsvorrichtung zur Erzeugung eines radiologischen 3D-Bilddatensatzes,
**dadurch gekennzeichnet, dass** es zur Durchführung eines Verfahrens nach einem der Ansprüche 1 bis 4 ausgebildet ist.

6. Medizinische Bildgebungsvorrichtung nach Anspruch 5,
**dadurch gekennzeichnet, dass** die Bildgebungsvorrichtung als Computertomographiegerät mit einer kippbaren, drehbaren und/oder verschiebbaren Gantry und/oder mit einer ausgebildet kippbaren, drehbaren und/oder verschiebbaren Patientenhalterungsvorrichtung, insbesondere Patientenliege, ausgebildet ist.

7. Verfahren zur Erstellung eines Therapieplans für die Strahlentherapie, insbesondere zum Bestrahlen eines Patienten mit Partikeln einer Partikeltherapieanlage, mit folgenden Verfahrensmerkmalen:
- Erzeugen eines artefaktreduzierten radiologischen Bilddatensatzes mit einem Verfahren nach einem der Ansprüche 1 bis 4,
- Planen von Bestrahlungsparametern, wie Bestrahlungsbereich, Dosisverteilung, Partikelstrahlenergie und/oder Partikelstrahlintensität mit dem artefaktreduzierten radiologischen Bilddatensatz als Grundlage.
